# EUROPÄISCHE PATENTSCHRIFT

(11) **EP 2 812 073 B1**
(45) Veröffentlichungstag und Bekanntmachung des Hinweises auf die Patenterteilung: **27.06.2018**
(21) Anmeldenummer: 13704356.8
(22) Anmeldetag: 08.02.2013
(51) Int. Cl.: A61P 7/08, A61M 1/16, A61M 1/14, A61K 45/06, A61K 47/32, A61K 9/08, A61K 31/19, A61K 33/00, A61K 33/06, A61K 33/14, A61K 33/42

(54) **KONZENTRAT FÜR EINE DIALYSIERFLÜSSIGKEIT UND DARAUS HERGESTELLTE DIALYSIERFLÜSSIGKEIT**
CONCENTRATE FOR A DIALYSIS FLUID AND DIALYSIS FLUID PRODUCED THEREFROM
CONCENTRAT POUR UN LIQUIDE DE DIALYSE ET LIQUIDE DE DIALYSE PRÉPARÉ À PARTIR DE CELUI-CI

(30) Priorität: 08.02.2012 DE 102012002372; 08.02.2012 US 201261596271 P
(43) Veröffentlichungstag der Anmeldung: 17.12.2014
(73) Patentinhaber: Fresenius Medical Care Deutschland GmbH, 61352 Bad Homburg (DE)
(72) Erfinder: TERPIN, Andreas, 60385 Frankfurt am Main (DE); NIER, Volker, 61203 Reichelsheim (DE)
(74) Vertreter: Ricker, Mathias
(86) Internationale Anmeldenummer: PCT/EP2013/000388
(87) Internationale Veröffentlichungsnummer: WO 2013/117345

(56) Entgegenhaltungen:
- EP-A1- 0 046 971
- EP-A2- 0 064 393
- WO-A1-00/51704
- WO-A1-00/57935
- WO-A1-2011/037983
- WO-A2-2007/016377
- DE-A1- 2 512 212
- GB-A- 2 417 420
- US-A- 3 669 878
- Pierce Biotechnology: "TECH TIP #62", , 1 January 2007 (2007-01-01), XP055231297, Retrieved from the Internet: URL:https://www.thermofisher.com.au/Upload s/file/Scientific/Applications/Life-Scienc e-Research-Technologies/Ion-exchange-chrom atography-(Tech-Tip-62).pdf [retrieved on 2015-11-25]
- Dowex Dow Corp.: "DOWEX MAC-3 A Macroporous Weak Acid Cation Exchange Resin used for Water Softening, Dealkalization & Demineralization Applications Product Information Guaranteed Sales Specifications H + form Product Type Matrix Functional group Recommended Operating Conditions DOWEX Ion Exchange Resins", , 1 January 1998 (1998-01-01), XP055340626, Retrieved from the Internet: URL:http://www.cst.rs/pdf/mac-3.pdf [retrieved on 2017-01-31]

## Beschreibung

Die vorliegende Erfindung betrifft ein Konzentrat für eine Dialysierflüssigkeit welche aus dem Konzentrat hergestellt und zur Blutreinigung verwendet werden kann. Die Erfindung betrifft ferner ein Verfahren zur Herstellung der Dialysierflüssigkeit, die Verwendung einer Dialysevorrichtung zur Herstellung einer Dialysierflüssigkeit, sowie eine Dialysevorrichtung, welche das Konzentrat oder die Dialysierflüssigkeit aufweist.

Bekanntlich wird bei der Blutreinigung durch Hämodialyse nach dem Prinzip des Konzentrationsausgleichs kleinmolekularer Substanzen in zwei Flüssigkeiten verfahren, die durch eine semipermeable Membran voneinander getrennt sind. Auf der einen Seite befindet sich das zu reinigende Blut, welches Toxine, Elektrolyte sowie harnhaltige Substanzen wie Harnstoff und Harnsäure enthält. Auf der anderen Seite der Membran befindet sich eine Dialysierflüssigkeit, die die vom Blut abzutrennenden Stoffe aufnehmen soll. Die semipermeable Membran besitzt Poren, die die kleinen Moleküle wie Wasser, Elektrolyte und harnhaltige Substanzen durchlassen, aber große Moleküle wie Eiweiße und Blutzellen zurückhalten.

Typischerweise beträgt der Dialysatfluss, d.h. der Fluss an Dialysierflüssigkeit, ca. 0,5 l/min. Bei einer mittleren Dialysedauer von vier bis fünf Stunden werden demnach 120 l bis 150 l Dialysierflüssigkeit benötigt. Diese Menge wird i.A. nicht bevorratet sondern durch Mischen und Verdünnen eines basischen und eines sauren Konzentrats mit Wasser hergestellt, wobei das Mischen und Verdünnen üblicherweise in der Dialysevorrichtung erfolgt. Damit kann auch der pH-Wert der Dialysierflüssigkeit eingestellt werden. Zusätzlich können der Dialysierflüssigkeit in der Dialysevorrichtung je nach Behandlungserfordernis physiologisch verträgliche Elektrolyte und gegebenenfalls Zucker wie Glukose zugemischt werden.

Basische Konzentrate für Dialysierlösungen bestehen im Allgemeinen hauptsächlich aus Natriumbikarbonat und werden zumeist in trockener Form, den sogenannten Trockenkonzentraten, geliefert. Saure Konzentrate werden häufig in flüssiger Form geliefert, da die Auswahl an physiologisch verträglichen Säuren, die in fester Form vorliegen, eingeschränkt ist. Meist werden in flüssigen Säurekonzentraten Salzsäure oder Essigsäure verwendet, da bei entsprechender Verdünnung die entsprechenden Anionen für die Verträglichkeit unproblematisch sind.

Die Verwendung von Flüssigkonzentraten bedingt, dass mit den für die Dialysierflüssigkeit benötigten Elektrolyten erhebliche Mengen an Wasser transportiert und gelagert werden müssen. Dies ist für Personal und Logistik ungünstig.

Flüssige Säurekonzentrate und deren Anwendung in der Hämodialyse zusammen mit einem Bikarbonatpuffer werden zum Beispiel in der EP 0 457 960 A2 beschrieben.

Aus der US-A-5,252,213 sind Säurekonzentrate in fester Form bekannt. Dabei wird hauptsächlich Zitronensäure als feste Säure eingesetzt. Allerdings ist auch bekannt, dass Zitronensäure nur in einem beschränkten Konzentrationsbereich eingesetzt werden kann, da sie die Gerinnung von Blut beeinflussen kann. Es ist ferner bekannt, dass Säuren mit der in vielen Dialysierflüssigkeiten enthaltenen Glukose Wechselwirkungen eingehen können, die zu einer geringeren Haltbarkeit entsprechender Trockenkonzentrate führen kann.

Die WO00/51704 A1 offenbart zweiteilige Vorrichtungen zur Herstellung von Lösungen zur Peritonealdialyse, wobei einem Teil der Vorrichtung, welche Elektrolytsalze und andere Bestandteile enthält, eine Reinigungsvorrichtung vorgeschaltet ist, welche unter anderem einen Ionenaustauscher enthält.

Eine Aufgabe der vorliegenden Erfindung besteht in der Bereitstellung verbesserter Säurekonzentrate, vorzugsweise in Form von Trockenkonzentraten, zur Herstellung einer Dialysierflüssigkeit für die Blutreinigung.

Diese Aufgabe wird dadurch gelöst, dass im Konzentrat für eine Dialysierflüssigkeit, vorzugsweise ein Trockenkonzentrat, und in der Dialysierflüssigkeit ein saurer Ionenaustauscher als Säure verwendet wird.

Insbesondere die Verwendung eines erfindungsgemäßen Trockenkonzentrats bei der Dialyse von Blut ermöglicht eine Handhabung, die für Personal und Logistik günstig ist. Des Weiteren zeichnen sich die erfindungsgemäßen Trockenkonzentrate durch eine gute Lagerstabilität und Haltbarkeit aus.

Gemäß eines ***ersten Aspekts*** betrifft die vorliegende Erfindung ein Konzentrat geeignet für die Herstellung einer Dialysierflüssigkeit, vorzugsweise ein Trockenkonzentrat, zumindest aufweisend eine Säure und einen physiologisch verträglichen Elektrolyten, das dadurch gekennzeichnet ist, dass die Säure mindestens einen sauren Ionenaustauscher aufweist oder dass die Säure ein saurer Ionenaustauscher ist,
wobei der saure lonentauscher bei Einwirken von Wasser und/ oder .von Salzen, deren Kationen von den im lonentauscher vorhandenen Gruppen gebunden werden können, Protonen abgibt, und
wobei der saure Ionentauscher protonierte Gruppen aufweist, ausgewählt aus Sulfonat-, Sulfat-, Phosphat-, Phosphonat-, Carboxylatgruppen, wobei die Gruppen an ein vernetztes Polystyrol oder Poly(meth)acrylat gebunden sind; und
wobei der mindestens eine lonentauscher und der physiologisch verträgliche Elektrolyt zusammen, in einem Behälter vorliegen und
wobei der physiologisch verträgliche Elektrolyt Kationen aufweist, ausgewählt aus den Kationen des Magnesiums, Calciums, Kaliums, Natriums, oder aus Mischungen von zwei oder mehreren davon; und Anionen aufweist, ausgewählt aus Chlorid, Acetat, Lactat, oder zwei oder mehreren davon.

In einer Ausführungsform betrifft die vorliegende Erfindung ein Konzentrat für eine Dialysierflüssigkeit, vorzugsweise ein Trockenkonzentrat, welches aus einer Säure und einem physiologisch verträglichen Elektrolyten besteht, das dadurch gekennzeichnet ist, dass die Säure mindestens einen sauren Ionenaustauscher aufweist oder dass die Säure ein saurer Ionenaustauscher ist.

Dieser saure Ionenaustauscher kann bei Einwirken von Wasser und/oder von Salzen, deren Kationen von den im Ionenaustauscher vorhandenen Gruppen gebunden werden können, Protonen abgeben. Auf diese Weise kann der pH-Wert der Dialysierflüssigkeit kontrolliert und eingestellt werden.

In einer Ausführungsform kann der saure Ionenaustauscher zusammen mit den üblicherweise bei der Dialyse verwendeten Säuren verwendet werden, also beispielsweise zusammen mit Zitronensäure oder Salzsäure oder Essigsäure.

In einer bevorzugten Ausführungsform ist die im Konzentrat verwendete Säure mindestens ein saurer Ionenaustauscher, besteht also aus dem mindestens einen Ionenaustauscher.

Der Begriff *"saurer Ionenaustauscher"* wie in dieser Offenbarung verwendet, umfasst alle Substanzen, welche saure Gruppen aufweisen, und in denen Protonen durch lonenaustausch ersetzt werden können.

Erfindungsgemäß können unterschiedliche Arten von sauren Ionenaustauschern verwendet werden. In einer Ausführungsform liegt der saure Ionenaustauscher als Kationenaustauscher vor. In einer weiteren Ausführungsform weist der Kationenaustauscher vernetzte Kunstharze auf, vorzugsweise auf Basis von vernetzten Polymeren oder auf Zellulosebasis, die mit anionischen Gruppen, sogenannten kationenaktiven Gruppen, funktionalisiert sind.

Beispiele für vernetzte und ionenaustauschende Polymere sind Copolymerisate abgeleitet von Styrol, gegebenenfalls weiteren damit copolymerisierbaren Monomeren und mindestens einem Vernetzer, wie vorzugsweise Divinylbenzol, bei denen zumindest ein Teil der vorhandenen Benzolkerne mit kationenaktiven Gruppen funktionalisiert wird. Weitere Beispiele für vernetzte und ionenaustauschende Polymere sind vernetzte Poly(meth)acrylsäuren, die durch Copolymerisation von Vinylbenzol mit (Meth)Acrylsäureestern und anschließender Verseifung erhalten werden können.

Die Ionenaustauscher können in beliebigen Ausgestaltungen vorliegen. Bevorzugt werden Ionenaustauscher in fester Form, vorzugsweise in körniger Form oder in Pulverform verwendet.

Die Ionenaustauscher können auch in Form von lonenaustauschermembranen verwendet werden.

Die erfindungsgemäß verwendeten Ionenaustauscher sind aus dem Stand der Technik bekannt oder können nach bekannten Verfahren hergestellt werden. Der saure Ionenaustauscher weist protonierte und/ oder protonierbare Gruppen auf ausgewählt aus Sulfonat-, Sulfat-, Phosphat-, Phosphonat-, Carboxylatgruppen, oder aus zwei oder mehreren davon. In einer Ausführungsform sind diese Gruppen an ein vernetztes Polymer gebunden, vorzugsweise an vernetztes Polystyrol.

Der Begriff *"physiologisch verträglicher Elektrolyt"* wie hierin verwendet, umfasst alle Elektrolyte, die bei der Dialyse keine Unverträglichkeiten oder nachteilige Wirkungen im und am Patienten hervorrufen. Der physiologisch verträgliche Elektrolyt weist Kationen auf ausgewählt aus den Kationen des Magnesiums, Calciums, Kaliums, Natriums, oder aus Mischungen von zwei oder mehreren davon. Vorzugsweise werden dann die Anionen ausgewählt aus Chlorid, Acetat, Lactat, oder zwei oder mehreren dieser Anionen.

In einer Ausführungsform werden als physiologisch verträglicher Elektrolyt Salze verwendet ausgewählt aus: Natriumchlorid, Calciumchlorid, Magnesiumchlorid, Natriumlactat, Calciumlactat, Magnesiumlactat, Natriumacetat, Calciumacetat, Magnesiumacetat, oder aus Kombinationen aus zwei oder mehreren davon.

In einer weiteren Ausführungsform enthält das Konzentrat für die Dialysierflüssigkeit Kochsalz. Dies hat den Vorteil, dass bei Einwirken von Wasser auf den Ionenaustauscher und auf das Kochsalz Natriumionen in den sauren Ionenaustauscher gelangen und dort Protonen aus dem sauren Ionenaustauscher verdrängen können. Damit kann der pH-Wert der aus dem Konzentrat hergestellten Dialysierflüssigkeit gezielt eingestellt werden.

Demzufolge weist in einer Ausführungsform das erfindungsgemäße Konzentrat mindestens einen sauren Ionenaustauscher und einen physiologisch verträglichen Elektrolyten auf, wobei der physiologisch verträgliche Elektrolyt Kochsalz aufweist oder aus Kochsalz besteht; oder besteht das erfindungsgemäße Konzentrat aus mindestens einem sauren Ionenaustauscher und einem physiologisch verträglichen Elektrolyten, wobei der physiologisch verträgliche Elektrolyt Kochsalz aufweist oder aus Kochsalz besteht.

In einer bevorzugten Ausführungsform liegt das Konzentrat als Trockenkonzentrat vor.

Der Begriff *"Trockenkonzentrat"* wie hierin verwendet, bedeutet, dass im Konzentrat, wenn überhaupt, nur untergeordnete Mengen an Wasser vorliegen. Vorzugsweise ist der Wasseranteil geringer als 10 Gew.-%, weiter bevorzugt kleiner als 5 Gew.-% bezogen auf die Gesamtmenge an Konzentrat. Das Konzentrat, welches vorzugsweise in Form eines Trockenkonzentrats vorliegt, ist einteilig. In dieser Ausführungsform umfasst das Konzentrat oder besteht das Konzentrat aus einer Komponente. Der Begriff *"Komponente"* wie hierin verwendet, bedeutet dabei einen Bestandteil des Konzentrats. Bei einteiliger Form können der saure Ionenaustauscher und der physiologisch verträgliche Elektrolyt sowie weitere Bestandteile des Konzentrats, vorzugsweise Kochsalz sofern dies nicht im physiologisch verträglichen Elektrolyten enthalten ist, zusammen vorgelegt werden, vorzugsweise in Form einer Mischung.

In einer Ausführungsform betrifft die Erfindung ein Konzentrat für eine Dialysierflüssigkeit, vorzugsweise ein Trockenkonzentrat, zumindest aufweisend eine Säure und einen physiologisch verträglichen Elektrolyten, dadurch gekennzeichnet, dass die Säure mindestens einen sauren Ionenaustauscher aufweist oder ein saurer Ionenaustauscher ist, wobei der mindestens eine Ionenaustauscher und der physiologisch verträgliche Elektrolyt zusammen, vorzugsweise in Form einer Mischung, in einem Behälter vorliegen.

Der Begriff *"Behälter"* wie hierin verwendet schließt Begriffe wie *"Verpackung"* oder *"Beutel"* oder *"Kartusche"* ein. In einer Ausführungsform bedeutet der Begriff *"Behälter"* auch einen Behälter, der vorzugsweise aus einer Dialysevorrichtung oder einer Vorrichtung, welche mit einer Dialysevorrichtung verbunden ist, entfernt und in diese wieder eingesetzt werden kann. In einer weiteren Ausführungsform bedeutet der Begriff *"Behälter"* auch, dass dieser für Wasser und/oder eine physiologisch verträgliche Elektrolytlösung, vorzugsweise eine Kochsalzlösung, zugänglich ist, um die im Behälter befindlichen Komponenten zu lösen und/oder aus diesen Protonen freizusetzen.

In einer Ausführungsform kann der physiologisch verträgliche Elektrolyt weitere Zusatzstoffe wie vorzugsweise Glukose enthalten.

In einer weiteren Ausführungsform ist es auch möglich, den sauren Ionenaustauscher sowie Kochsalz und den physiologisch verträglichen Elektrolyten gemeinsam vorzulegen und eine basische Puffersubstanz, vorzugsweise Natriumhydrogencarbonat, getrennt davon.

In einer Ausführungsform kann der Ionenaustauscher nach der erfindungsgemäßen Verwendung und/oder nach Abgabe von Protonen wieder regeneriert werden. Dabei können dem Ionenaustauscher oder dem Behälter, in welchem der Ionenaustauscher vorliegt, vorzugsweise der Kartusche, vorzugsweise Mineralsäuren, wie Salzsäure, Schwefelsäure, Salpetersäure, oder Carbonsäuren wie Essigsäure, Zitronensäure oder Milchsäure zugeführt werden.
In einer weiteren Ausführungsform gemäß des ersten Aspekts der Erfindung kann das Konzentrat für eine Dialysierflüssigkeit nicht nur als Trockenkonzentrat vorliegen, sondern auch als Flüssigkonzentrat.

In einer Ausführungsform liegt dann das Konzentrat als Flüssigkonzentrat vor, welches neben dem sauren Ionenaustauscher und dem physiologisch verträglichen Elektrolyten Wasser aufweist, sowie vorzugsweise zusätzlich Kochsalz und optional Glukose.

Bevorzugte Konzentrate in Form von flüssigen Konzentraten enthalten:
(a) Wasser,
(b) den sauren Ionenaustauscher mit einer Austauscherkapazität für Kationen von 20 mEq/L bis 900 mEq/L,
(c) Chlorid in einer Konzentration von 1.000 mEq/L bis 7.000 mEq/L, vorzugsweise von 1.000 mEq/L bis 2.000 mEq/L, und
(d) wenigstens ein physiologisch annehmbares Kation und ein physiologisch annehmbares Anion.

In einer Ausführungsform weist das flüssige Konzentrat einen pH-Wert von kleiner als 4 auf.

Vorzugsweise wird als Wasser das bei der Dialyse üblicherweise verwendete Osmosewasser eingesetzt.

Gemäß eines ***dritten Aspekts*** betrifft die Erfindung ein Verfahren zur Herstellung der Dialysierflüssigkeit gemäß des zweiten Aspekts oder einer der darin beschriebenen Ausführungsformen, mindestens aufweisend die Stufe (i):
(i) Vermischen des Konzentrats gemäß des ersten Aspekts oder einer der darin beschriebenen Ausführungsformen mit Wasser.

In einer bevorzugten Ausführungsform des Verfahrens wird der saure Ionenaustauscher zusammen mit dem physiologisch verträglichen Elektrolyt und Kochsalz in einem Behälter vorgelegt, wobei das Konzentrat vorzugsweise als Trockenkonzentrat vorliegt. Nach Zugabe von Wasser werden durch das im Salzanteil vorhandene Kochsalz durch lonenaustausch die Protonen aus dem Ionenaustauscher freigesetzt.

Gemäß eines ***vierten Aspekts*** betrifft die Erfindung eine Dialysevorrichtung, mindestens aufweisend ein Konzentrat für eine Dialysierflüssigkeit gemäß des ersten Aspekts oder einer der darin beschriebenen Ausführungsformen, insbesondere der Ausführungsform, in welcher der saure Ionenaustauscher in einem Behälter vorliegt, wobei der Behälter in die Dialysevorrichtung oder in eine Vorrichtung eingebracht ist, welche mit der Dialysevorrichtung verbunden ist.

In einer Ausführungsform weist die Dialysevorrichtung weiter einen Behälter zum Aufnehmen von Kochsalz auf, welcher sich in der Dialysevorrichtung befindet oder in einer Vorrichtung, welche mit der Dialysevorrichtung verbunden ist, wobei beim Durchströmen des Behälters mit Wasser gelöstes Kochsalz in den den sauren Ionenaustauscher beherbergenden Behälter gelangt und dort aus dem sauren Ionenaustauscher Protonen freisetzt, die in die Dialysevorrichtung geleitet werden.

Gemäß eines ***sechsten Aspekts*** betrifft die Erfindung die Verwendung einer Dialysevorrichtung gemäß des vierten Aspekts oder einer der darin beschriebenen Ausführungsformen zur Durchführung eines Verfahrens gemäß des dritten Aspekts oder einer der darin beschriebenen Ausführungsformen
Gemäß eines ***siebten Aspekts*** betrifft die Erfindung die Verwendung eines sauren Ionenaustauschers:
- zur Herstellung eines Konzentrats für eine Dialysierflüssigkeit gemäß des ersten Aspekts; oder
- in einer Dialysiervorrichtung gemäß des vierten Aspekts oder in einer Vorrichtung, welche mit besagter Dialysevorrichtung verbunden ist.

### Beispiel:

Eine Dialysierflüssigkeit wird mittels eines sauren Ionenaustauschers so eingestellt, dass sie 3 mMol Protonen pro Liter enthält. Der saure Ionenaustauscher ist ein handelsüblicher saurer Ionenaustauscher auf Basis eines Carboxylgruppen-haltigen Acrylpolymeren mit einer lonenaustauschkapazität von 3.800 mMol Protonen pro Liter (Dowex® MAC-3). Für 120 Liter Dialysierflüssigkeit werden 95 ml des sauren Ionenaustauschers verwendet.

## Patentansprüche

1. Konzentrat geeignet für die Herstellung einer Dialysierflüssigkeit, zumindest aufweisend eine Säure und einen physiologisch verträglichen Elektrolyten, **dadurch gekennzeichnet, dass** die Säure mindestens einen sauren Ionenaustauscher aufweist oder ein saurer Ionenaustauscher ist,
wobei der saure Ionenaustauscher bei Einwirken von Wasser und/ oder von Salzen deren Kationen von den im lonentauscher vorhandenen Gruppen gebunden werden können, Protonen abgibt, und
wobei der saure lonentauscher protonierte Gruppen aufweist ausgewählt aus Sulfonat-, Sulfat-, Phosphat-, Phosphonat-, Carboxylatgruppen, wobei die Gruppen an ein vernetztes Polystyrol oder Poly(meth)acrylat gebunden sind; und
wobei der mindestens eine lonentauscher und der physiologisch verträgliche Elektrolyt zusammen, in einem Behälter vorliegen und
wobei der physiologisch verträgliche Elektrolyt Kationen aufweist ausgewählt aus den Kationen des Magnesiums, Calciums, Kaliums, Natriums, oder aus Mischungen von zwei oder mehreren davon; und Anionen aufweist ausgewählt aus Chlorid, Acetat, Lactat, oder zwei oder mehreren davon.

2. Konzentrat nach Anspruch 1, wobei der physiologisch verträgliche Elektrolyt Salze verwendet, ausgewählt aus: Natriumchlorid, Calciumchlorid, Magensiumchlorid, Natriumlactat, Calciumlactat, Magnesiumlactat, Natriumacetat, Calciumacetat, Magnesiumacetat, oder Kombinationen aus zwei oder mehreren davon.

3. Konzentrat nach einem der vorstehenden Ansprüche 1 oder 2, **dadurch gekennzeichnet, dass** der Elektrolyt Kochsalz aufweist

4. Konzentrat nach Anspruch 3, **dadurch gekennzeichnet, dass** der Elektrolyt aus Kochsalz besteht.

5. Konzentrat nach einem der vorstehenden Ansprüche, **dadurch gekennzeichnet, dass** der saure lonentauscher und der physiologisch verträgliche Elektrolyt in Form einer Mischung vorliegen.

6. Konzentrat nach einem der vorstehenden Ansprüche, wobei es als Trockenkonzentrat vorliegt.

7. Verfahren zur Herstellung einer Dialysierflüssigkeit, mindestens aufweisend die Stufe (i):
(i) Vermischen des Konzentrats nach einem der Ansprüche 1 bis 6 mit Wasser.

8. Dialysevorrichtung, mindestens aufweisend ein Konzentrat nach einem der Ansprüche 1 bis 6 geeignet für die Herstellung einer Dialysierflüssigkeit, wobei das Konzentrat in einem Behälter in die Dialysevorrichtung oder in eine Vorrichtung eingebracht ist, welche mit der Dialysevorrichtung verbunden ist.

9. Verwendung einer Dialysevorrichtung nach Anspruch 8 zur Durchführung eines Verfahrens nach Anspruch 7.

10. Verwendung eines sauren Ionenaustauschers, wie in Anspruch 1 definiert
- zur Herstellung eines Konzentrats für eine Dialysierflüssigkeit nach einem der Ansprüche 1 bis 6; oder
- in einer Dialysiervorrichtung nach Anspruch 8 oder in einer Vorrichtung, welche mit besagter Dialysevorrichtung verbunden ist.

## Claims

1. A concentrate suitable for the preparation of a dialysis fluid, at least comprising an acid and a physiologically acceptable electrolyte, **characterized in that** the acid comprises at least one acidic ion exchanger or is an acidic ion exchanger,
wherein the acidic ion exchanger, upon the action of water and / or of salts whose cations can be bound by the groups present in the ion exchanger, releases protons, and
wherein the acidic ion exchanger comprises protonated groups selected from sulfonate, sulfate, phosphate, phosphonate, carboxylate groups, wherein the groups are bound to a crosslinked polystyrene or poly(meth)acrylate; and
wherein the at least one ion exchanger and the physiologically acceptable electrolyte are present together in a container and
wherein the physiologically acceptable electrolyte comprises cations selected from the cations of magnesium, calcium, potassium, sodium, or mixtures of two or more thereof; and comprises anions selected from chloride, acetate, lactate, or two or more thereof.

2. The concentrate according to claim 1, wherein the physiologically acceptable electrolyte uses salts selected from: sodium chloride, calcium chloride, magnesium chloride, sodium lactate, calcium lactate, magnesium lactate, sodium acetate, calcium acetate, magnesium acetate, or combinations of two or more thereof.

3. The concentrate according to any one of the preceding claims 1 or 2, **characterized in that** the electrolyte comprises sodium chloride.

4. The concentrate according to claim 3, **characterized in that** the electrolyte consists of sodium chloride.

5. The concentrate according to any one of the preceding claims, **characterized in that** the acidic ion exchanger and the physiologically acceptable electrolyte are present in the form of a mixture.

6. The concentrate according to any one of the preceding claims, wherein it is present as a dry concentrate.

7. A method for producing a dialysis fluid, at least comprising step (i):
(i) mixing the concentrate according to any one of claims 1 to 6 with water.

8. A dialysis device, at least comprising a concentrate according to any one of claims 1 to 6 suitable for the preparation of a dialysis fluid, wherein the concentrate is introduced in a container into the dialysis device or into a device which is connected to the dialysis device.

9. A use of a dialysis device according to claim 8 for carrying out a method according to claim 7.

10. A use of an acidic ion exchanger as defined in claim 1
- for the preparation of a concentrate for a dialysis fluid according to any one of claims 1 to 6; or
- in a dialysis device according to claim 8 or in a device connected to said dialysis device.

## Revendications

1. Concentrat approprié pour la préparation d'un liquide de dialyse, comportant au moins un acide et un électrolyte physiologiquement acceptable, **caractérisé en ce que** l'acide présente au moins un échangeur d'ions acides ou est un échangeur d'ions acides,
dans lequel l'échangeur d'ions acides cède des protons sous l'action de l'eau et/ou de sels, dont les cations peuvent être liés aux groupes présents dans l'échangeur d'ions, et
dans lequel l'échangeur d'ions acide comporte des groupes protonés choisis parmi les groupes sulfonate, sulfate, phosphate, phosphonate, carboxylate, dans lequel les groupes sont liés à un polystyrène réticulé ou un poly(méth)acrylate ; et
dans lequel ledit au moins un échangeur d'ions et l'électrolyte physiologiquement acceptable sont présents conjointement dans un récipient et
dans lequel l'électrolyte physiologiquement acceptable présente des cations choisis parmi les cations de magnésium, de calcium, de potassium, de sodium, ou des mélanges de deux de ceux-ci ou plus ; et présente des anions choisis parmi le chlorure, l'acétate, le lactate, ou deux de ceux-ci ou plus.

2. Concentrat selon la revendication 1, dans lequel l'électrolyte physiologiquement acceptable utilise des sels choisis parmi : le chlorure de sodium, le chlorure de calcium, le chlorure de magnésium, le lactate de sodium, le lactate de calcium, le lactate de magnésium, l'acétate de sodium, l'acétate de calcium, l'acétate de magnésium ou des combinaisons de deux de ceux-ci ou plus.

3. Concentrat selon l'une des revendications précédentes 1 ou 2, **caractérisé en ce que** l'électrolyte présente du chlorure de sodium.

4. Concentrat selon la revendication 3, **caractérisé en ce que** l'électrolyte se compose de chlorure de sodium.

5. Concentrat selon l'une des revendications précédentes, **caractérisé en ce que** l'échangeur d'ions acides et l'électrolyte physiologiquement acceptable se présentent sous la forme d'un mélange.

6. Concentrat selon l'une des revendications précédentes, dans lequel il se présentant sous forme de concentrat sec.

7. Procédé de préparation d'un liquide de dialyse, présentant au moins l'étape (i) :
(i) mélange du concentrat selon l'une des revendications 1 à 6 avec de l'eau.

8. Appareil de dialyse, présentant au moins un concentrat selon l'une des revendications 1 à 6, approprié pour la préparation d'un liquide de dialyse, dans lequel le concentrat se trouvant dans un récipient est introduit dans l'appareil de dialyse ou dans un dispositif qui est relié à l'appareil de dialyse.

9. Utilisation d'un appareil de dialyse selon la revendication 8 pour la réalisation d'un procédé selon la revendication 7.

10. Utilisation d'un échangeur d'ions, tel que défini selon la revendication 1
- pour la préparation d'un concentrat pour un liquide de dialyse selon l'une des revendications 1 à 6 ; ou
- dans un appareil de dialyse selon la revendication 8 ou dans un dispositif qui est relié audit appareil de dialyse.
